# EUROPÄISCHE PATENTSCHRIFT

(11) **EP 2 039 324 B1**
(45) Veröffentlichungstag und Bekanntmachung des Hinweises auf die Patenterteilung: **02.11.2011**
(21) Anmeldenummer: 07018277.9
(22) Anmeldetag: 18.09.2007
(51) Int. Cl.: A61F 2/16

(54) **Intraokularlinse**
Intraocular lens
Lentille intraoculaire

(43) Veröffentlichungstag der Anmeldung: 25.03.2009
(73) Patentinhaber: Amon, Michael, 1090 Wien (AT)
(72) Erfinder: Amon, Michael, Prof., Dr., 1090 Wien (AT); Serester, Alexander, Dipl.-Ing.,, 93077 Bad Abbach (DE)
(74) Vertreter: Eisenführ, Speiser & Partner

(56) Entgegenhaltungen:
- WO-A-02/09619
- US-A- 4 485 498
- US-A- 5 171 320
- US-A- 5 476 512

## Beschreibung

Die Erfindung betrifft eine Intraokularlinse nach den Oberbegriff des Patentanspruches 1.

Eine derartige Intraokularlinse ist aus WO 02/09619 A bekannt. Die bekannte Intraokularlinse beinhaltet einen aus einem optischen Linsenteil und einem haptischen Linsenteil bestehenden Linsenkörper, wobei der haptische Linsenteil zwei von Verbindungsstellen an der Oberfläche des optischen Linsenteils sich wegerstreckende Haptikbügel aufweist. Um den gesamten Linsenumfang erstreckt sich eine Eingriffsstelle, insbesondere in Form einer umlaufenden Nut, welche den die Kapsulorhexis umgebenden Randbereich der eröffneten Linsenkapsel bei der Implantation aufnimmt.

Aufgabe der Erfindung ist es, eine Intraokularlinse der eingangs genannten Art zu schaffen, welche mit verbesserter Lagestabilität einfach implantiert werden kann.

Diese Aufgabe wird durch die Merkmale des Patentanspruches 1 gelöst. Die Unteransprüche beinhalten vorteilhafte Weiterbildungen der Erfindung.

In bevorzugter Weise sind am Linsenkörper zwei diametral zueinander liegende Eingriffsstellen, insbesondere im Bereich der Verbindungsstellen vorgesehen. Die Eingriffsstellen können hierzu am optischen Linsenteil vorgesehen sein. Die jeweilige Eingriffsstelle kann entsprechend ihrer oben beschriebenen Eigenschaft als Stufe, als Nut, als Hinterschneidung, als Kerbe oder dergleichen ausgebildet sein.

In bevorzugter Weise liegen die Eingriffsstellen außerhalb einer durch den äußeren Umfangsrand des optischen Linsenteils gelegten Mittelebene. Hierbei können die Eingriffsstellen an der einen Seite der Mittelebene und auf der anderen Seite der Mittelebene können am optischen Linsenteil Oberflächenteile vorhanden sein, welche zum Anlegen des restlichen Teils des die Kapsulorhexis umgebenden Randbereichs der Linsenkapsel geeignet sind. In bevorzugter Weise befinden sich die Eingriffsstellen und die am optischen Linsenteil vorhandenen Oberflächenteile, welche zum Anlegen des restlichen Teils des die Kapsulorhexis umgebenden Randbereichs der Linsenkapsel geeignet sind, bezüglich der Mittelebene auf der gleichen Seite. Hierdurch wird erreicht, dass die Teile der Kapsulorhexis, welche in den Eingriffsstellen liegen, und die Teile der Kapsulorhexis, welche auf dem optischen Linsenteil aufliegen, etwa in der gleichen Ebene oder in Ebenen, welche einen nur geringen Abstand voneinander aufweisen, liegen.

Auf diese Weise erreicht man mit Hilfe des die Kapsulorhexis umgebenden Randbereichs der Linsenkapsel eine zusätzliche Fixierung und Positionierung der Intraokularlinse im Auge. Ferner liegt der eröffnete Teil der Linsenkapsel, nämlich entweder die hintere Linsenkapsel oder die vordere Linsenkapsel mit mechanischer Spannung mit ihrem die Kapsulorhexis umgebenden Randbereich am Linsenkörper an.

Gemäß Patentanspruch 14 kann die Intraokularlinse als gedrehte Intraokularlinse ausgebildet sein, wobei die plattenförmig ausgebildeten Haptikbügel und der optische Linsenteil als gedrehter Linsenkörper einstückig ausgebildet sind. Diese Linse kann aus einem Linsenrohling, insbesondere mit Hilfe eines Diamantdrehwerkzeugs gedreht werden. Bei einer derartig gedrehten Intraokularlinse ist es nicht erforderlich, den Linsenkörper in einem zusätzlichen Poliervorgang an der Oberfläche zu polieren.

Die so hergestellte Intraokularlinse weist die Besonderheit auf, dass Übergangsflächen zwischen dem optischen Linsenteil und den plattenförmig ausgebildeten Haptikbügeln teilzylindrische (torische) Oberflächen aufweisen. Hierdurch wird ein kontinuierlicher Übergang zwischen den plattenförmigen Haptikbügeln und der insbesondere sphärisch ausgebildeten Oberfläche des optischen Linsenteils erreicht. Auch bei asphärisch ausgebildeten Oberflächen des optischen Linsenteils lässt sich mit Hilfe der teilzylindrischen (torischen) Übergangsbereich zwischen dem optischen Linsenteil und den plattenförmigen Abdeckbügeln ein kontinuierlicher Übergang erreichen.

Die Zylinderachsen der teilzylindrischen Oberflächen verlaufen parallel zu einer durch die Mitte des Linsenkörpers gelegten Mittellinie.

Anhand der Figuren wird an Ausführungsbeispielen die Erfindung noch näher erläutert.

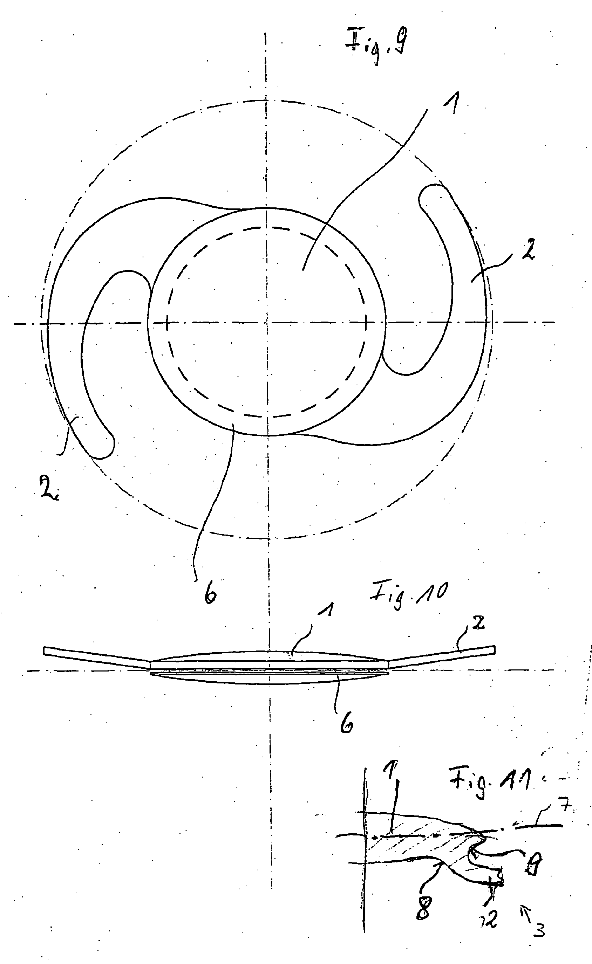

Es zeigt
- Fig. 1: ein erstes Ausführungsbeispiel in Draufsicht;
- Fig. 2: das Ausführungsbeispiel der Fig. 1 in Seitenansicht;
- Fig. 3: ein zweites Ausführungsbeispiel in Draufsicht;
- Fig. 4: das Ausführungsbeispiel der Fig. 3 in Seitenansicht;
- Fig. 5: ein drittes Ausführungsbeispiel in Draufsicht;
- Fig. 6: das Ausführungsbeispiel der Fig. 5 in Seitenansicht;
- Fig. 7: ein viertes Ausführungsbeispiel in Draufsicht;
- Fig. 8: das Ausführungsbeispiel der Fig. 7 in Seitenansicht;
- Fig. 9: ein fünftes Ausführungsbeispiel in Draufsicht;
- Fig. 10: das Ausführungsbeispiel der Fig. 9 in Seitenansicht;
- Fig. 11: eine Detaildarstellung einer Verbindungsstelle zwischen optischem Linsenteil und Haptikbügel beim Ausführungsbeispiel der Fig. 1 und 2;
- Fig. 12:
- bis 14: ein Ausführungsbeispiel für eine Intraokularlinse mit den in Fig. 11 gezeigten Verbindungsstellen zwischen Haptik und optischem Linsenteil.

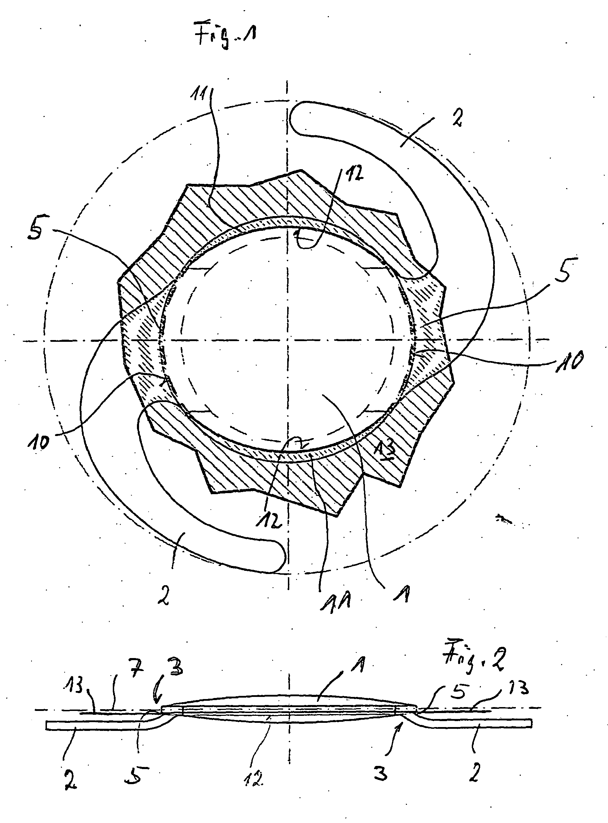

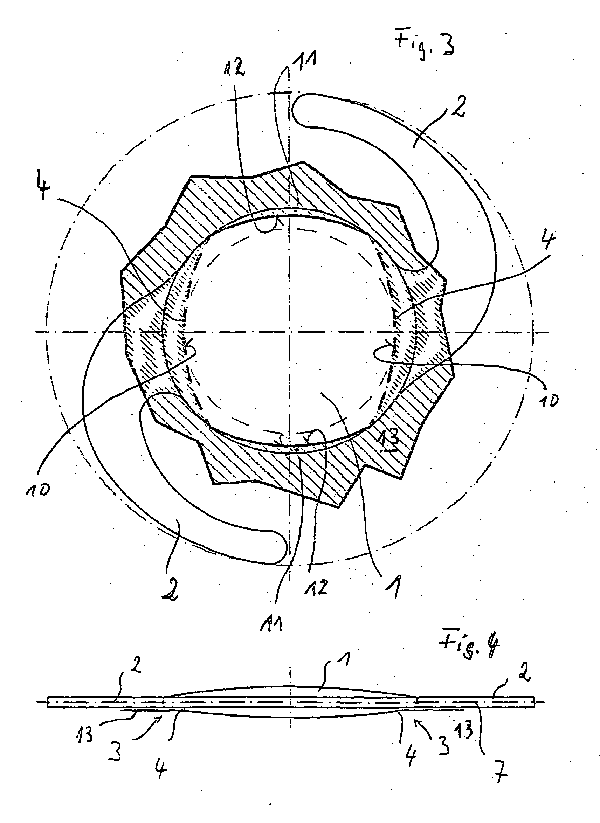

Die dargestellten Ausführungsbeispiele von erfindungsgemäßen Intraokularlinsen beinhalten jeweils einen optischen Linsenteil 1 und einen haptischen Linsenteil, welcher zwei von Verbindungsstellen 3 am Umfang des optischen Linsenteils sich wegerstreckende Haptikbügel 2 aufweist. Bei den Ausführungsformen der Fig. 1 bis 4 und 9, 10 sind die Haptikbügel in Draufsicht als flache, plattenförmige Bügel ausgebildet, die aus einem Stück mit dem Linsenkörper 1 gebildet sind. Bei den Ausführungsbeispielen der Fig. 1 und 2 erstrecken sich die Haptikbügel 2 außerhalb einer Mittelebene 7 des Linsenkörpers und parallel zu dieser Mittelebene und beim Ausführungsbeispiel der Fig. 3 verläuft die Mittelebene 7 durch die Haptikbügel 2. Die Mittelebene 7 des Linsenkörpers durchsetzt den äußeren Umfangsrand des optischen Linsenteils 1. Beim Ausführungsbeispiel der Fig. 9 und 10 verlaufen die Haptikbügel 2 schräg geneigt zur Mittelebene 7.

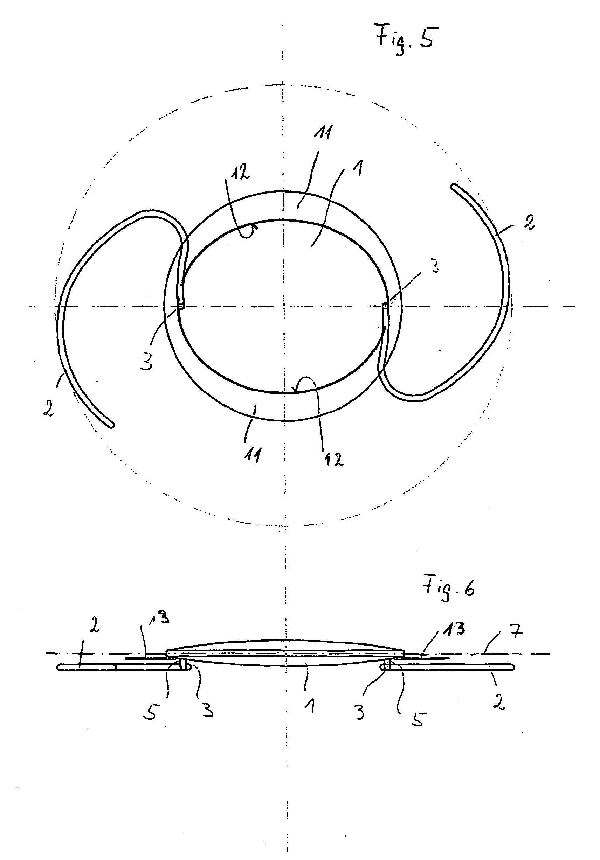

Bei den Ausführungsbeispielen der Fig. 5 bis 8 sind die Haptikbügel 2 fadenförmig ausgebildet und erstrecken sich parallel und außerhalb der Mittelebene 7. Es ist jedoch auch möglich, die fadenförmigen Haptikbügel schräg in einem spitzen Winkel gegenüber der Mittelebene, wie beim Ausführungsbeispiel der Fig. 9 und 10 anzuordnen.

Die in den Figuren dargestellten erfindungsgemäßen Ausführungsbeispiele besitzen zumindest im Bereich der Verbindungsstellen 3 zwischen dem Linsenkörper 1 und den Haptikbügeln 2 Eingriffsstellen, an denen zumindest ein Teil des eine Kapsulorhexis umgebenden Randbereichs einer Linsenkapselwand 13 aufgenommen werden kann. Diese Kapsulorhexis kann in der vorderen Linsenkapsel und der hinteren Linsenkapsel für die Entnahme einer eingetrübten natürlichen Augenlinse gelegt worden sein.

Beim Ausführungsbeispiel der Fig. 1 und 2 sind die Eingriffsstellen als zwischen den Haptikbügeln und dem optischen Linsenteil 1 liegende Nuten 5 oder Kerben ausgebildet. Diese Eingriffsstellen liegen diametral am optischen Linsenteil 1 und erstrecken sich über die Breite der Verbindungsstellen 3 im Übergangsbereich zwischen den Haptikbügeln 2 und dem optischen Linsenteil 1.

Bei der Implantation der Intraokularlinse greifen innenliegende die Kapsulorhexis umfassende Kapselrandbereiche mit innen liegenden Rändern 10 in die diametral liegenden Nuten 5 ein. Diese in den Nuten 5 liegenden Ränder 10 der Kapsulorhexis sind in der Fig. 1 strichliert dargestellt.

Die verbleibenden Ränder 12 der Kapsulorhexis liegen auf der Oberfläche des optischen Linsenteils 1 auf, welche auf der gleichen Seite der Mittellinie 7 liegt. An die Ränder 12 schließen sich etwa sichelförmige Kapselrandbereiche 11 einer Linsenkapselwand 13 an, welche ebenfalls auf der Oberfläche des optischen Linsenteils 1 liegen. Die Linsenkapselwand 13 kann die vordere (Vorderkapsel) oder hintere (Hinterkapsel) Kapselwand der Linsenkapsel sein.

Bei dem in den Fig. 3 und 4 dargestellten Ausführungsbeispiel sind die diametral liegenden Eingriffsstellen als Stufen 4 am Linsenkörper und insbesondere am optischen Linsenteil 1 ausgebildet. Die Stufen 4 liegen ebenfalls an diametral liegenden Stellen im Bereich der Verbindungsstellen 3 zwischen den Haptikbügeln und dem optischen Linsenteil 3. Beim Implantieren der Intraokularlinse liegen die in Fig. 3 strichliert dargestellten Ränder 10 der Kapsulorhexis in den Stufen 4. Dabei liegen sie auf einer Seite der Mittellinie 7 des Linsenkörpers. Die restlichen Ränder 12 der Kapsulorhexis liegen auf der Oberfläche des optischen Linsenteils 1, welche auf der anderen Seite der Mittellinie 7 sich befindet, auf. Diese Ränder 12 sind in der Fig. 3 mit durchgezogenen Linien dargestellt. Angrenzende sichelförmige Kapselrandbereiche 11 liegen ebenfalls auf der Oberfläche des optischen Linsenteils 1 auf, wie aus Fig. 3 zu ersehen ist.

In den Fig. 1 und 3 ist die Kapsulorhexis im wesentlichen kreisrund mit glatten Begrenzungsrändern 10 und 12 dargestellt. In der Praxis ist der die Kapsulorhexis umgebende Innenrand der Linsenkapsel nur annähernd kreisrund und kann einen gezackten Verlauf aufweisen.

Beim dargestellten Ausführungsbeispiel der Fig. 5 und 6 sind fadenförmige Haptikbügel 2 vorgesehen. Beim dargestellten Ausführungsbeispiel besitzt jeder Haptikbügel 2 eine Verbindungsstelle 3 mit dem optischen Linsenteil. Es ist jedoch auch möglich, eine oder mehrere zusätzliche Verbindungsstellen in dem parallel mit dem Umfangsrand des optischen Linsenteils verlaufenden Bügelteil vorzusehen. Im Bereich dieser Verbindungsstellen werden zwischen dem jeweiligen Bügel 2 und dem optischen Linsenteil Kerben oder Nuten 5 gebildet, mit denen Innenränder entsprechend den Innenrändern 10 in den Ausführungsbeispielen der Fig. 1 bis 4 der die Kapsulorhexis umgebenden Kapselrandbereiche der Kapselwand 13 (Fig. 6) in Eingriff kommen. Diese Randbereiche liegen, wie aus Fig. 6 zu ersehen ist, auf der einen Seite (Unterseite) der Mittellinie 7 des Linsenkörpers. Die beiden übrigen, den Rändern 12 in den Fig. 1 und 3 entsprechenden Ränder mit sich anschließenden sichelförmigen Randbereichen 11 liegen auf der gleichen Seite (Unterseite) der Mittellinie 7 auf der Oberfläche des optischen Linsenteils 1 auf.

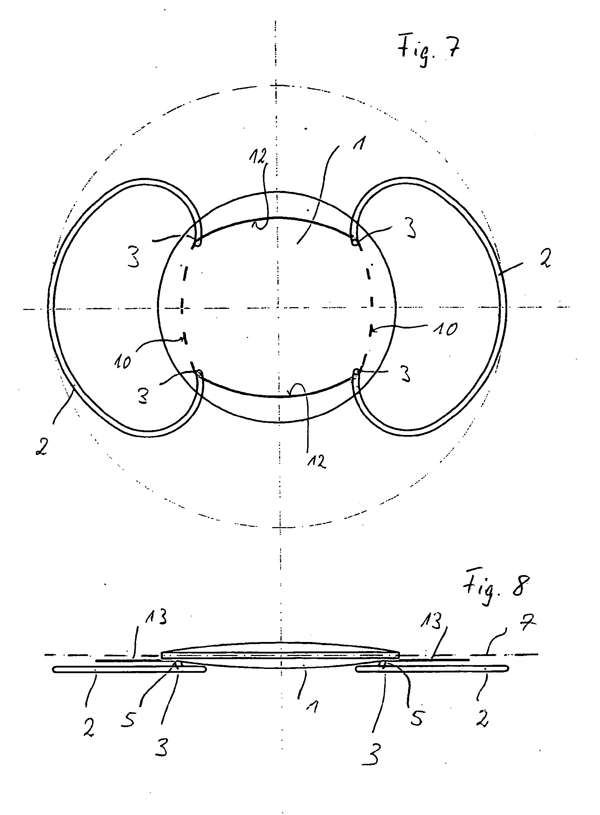

Beim Ausführungsbeispiel der Fig. 7 und 8 sind die fadenförmigen Haptikbügel 2 an ihren beiden Enden mit dem optischen Linsenteil 1 in den Verbindungsstellen 3 verbunden. Wie beim Ausführungsbeispiel der Fig. 5 und 6, werden in den Bereichen der Verbindungsstellen Nuten bzw. Kerben 5 gebildet, mit denen die entsprechenden Ränder 10 der Kapsulorhexis auf der einen Seite der Mittellinie 7 zum Eingriff kommen. Die anderen Ränder entsprechen den Rändern 12 in den Fig. 1 und 3 mit den sich anschließenden sichelförmigen Kapselrandbereichen 11 liegen auf der gleichen Seite der Mittellinie 7 auf der Oberfläche des optischen Linsenteils 1 auf.

Bei dem in den Fig. 9 und 10 dargestellten Ausführungsbeispiel ist eine umlaufende Kerbe oder Nut 6 vorgesehen. Diese erstreckt sich etwa im Bereich der Mittellinie 7 des Linsenkörpers. Bei diesem Ausführungsbeispiel kann der gesamte die Kapsulorhexis umgebende Rand der Linsenkapsel in die Nut 6 eingelegt werden. Es ist jedoch auch möglich, die Nut 6 im Bereich der Verbindungsstellen 3, an denen die Haptikbügel 2 mit dem optischen Linsenteil verbunden sind, vorzusehen und bei der Implantation die Randbereiche, welche die Kapsulorhexis umgeben, in der Weise in den Nuten 6 und auf der Oberfläche des optischen Linsenteils 1 anzuordnen, wie es bei den vorher beschriebenen Ausführungsbeispielen der Fall ist. Anstelle der umlaufenden Nut 6 kann auch eine anders ausgebildete umlaufende Eingriffsstelle, beispielsweise in Form einer umlaufenden Stufe vorgesehen sein.

In Fig. 11 ist schnittbildlich der Bereich einer Verbindungsstelle 3 des in den Fig. 1 und 2 dargestellten Ausführungsbeispiels vergrößert dargestellt. Hieraus ergibt sich, dass eine Übergangsfläche 8 zwischen dem optischen Linsenteil 1 und dem jeweiligen Haptikbügel 2 torisch, insbesondere zylindrisch torisch ausgebildet ist. Hierdurch vereinfacht sich die Herstellung des Linsenkörpers aus einem Stück. Eine dabei entstandene Übergangsfläche 9 bildet zwischen dem optischen Linsenteil und dem Haptikbügel die Kerbe bzw. Nut 5.

Beim Einfügen des Linsenkörpers in eine Kapsulorhexis in der Vorderkapsel weist der Linsenkörper vorzugsweise Haptikbügel auf, die zur Abstützung im Sulcus des Auges geeignet sind. Bei einem Einsetzen des Linsenkörpers in eine Kapsulorhexis in der Hinterkapsel sind die Haptikbügel vorzugsweise zur Abstützung im Kapselsack, insbesondere am Äquator des Kapselsackes geeignet.

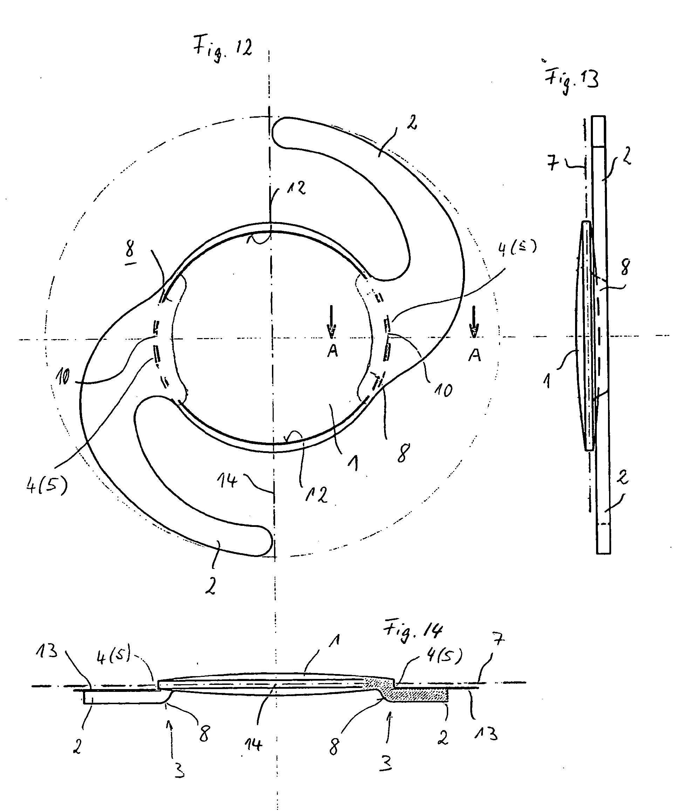

Das in den Fig. 12 bis 14 dargestellte Ausführungsbeispiel weist in den Übergangsbereichen zwischen den plattenförmigen Haptikbügeln 2 und dem optischen Linsenteil 1 Oberflächen 8 auf, welche, wie bei Fig. 11 schon erläutert, teilzylindrisch (torisch) ausgebildet sind. Die Zylinderachsen dieser teilzylindrischen Oberflächen 8 verlaufen im wesentlichen parallel zu einer durch die Linsenmitte gelegten Mittellinie 14. Der übrige Teil der Oberfläche des optischen Linsenteils 1 kann sphärisch oder asphärisch ausgebildet sein. Wie insbesondere aus der Fig. 14 zu ersehen ist, ergibt sich dabei ein kontinuierlicher Übergang zwischen der Haptik und der Oberfläche des optischen Linsenteils 1. Diese Eigenschaft wird auch beim Ausführungsbeispiel der Fig. 1 und 2 erreicht. Vorzugsweise werden die Linsenkörper der in den Fig. 1 und 2 sowie 12 bis 14 gezeigten Ausführungsbeispiele als gedrehte Linsenkörper ausgebildet, die einstückig mit Drehen aus einem Linsenrohling gewonnen wird. Sowohl die plattenförmigen Haptikbügel 2 als auch der optische Linsenteil 1 bestehen somit aus einem einstückigen Linsenkörper.

In der Fig. 12 ist eine Draufsicht auf das Ausführungsbeispiel gezeigt. In der Fig. 13 ist eine Seitenansicht von der rechten Seite in der Fig. 12 gezeigt. In der Fig. 14 ist ebenfalls eine Seitenansicht mit Blickrichtung von unten nach oben in der Zeichenebene der Fig. 12 gezeigt. Ferner ist in der Fig. 14 ein Teilschnitt entlang einer Schnittlinie A-A in Fig. 12 dargestellt.

Der Innenrand einer Kapsulorhexis in einer Linsenkapselseitenwand 13 liegt in einer Stufe 4 oder in einer Nut 5 mit beispielsweise der in Fig. 11 gezeigten Übergangsfläche 9 an. Die Stufen 4 bzw. Nuten 5 liegen, wie beim Ausführungsbeispiel der Fig. 1 und 2 diametral einander gegenüber. Die Kapsulorhexis in der Linsenkapselwand 13 liegt mit dem Randbereich 10 an der Stufe 4 bzw. in der Nut 5 an. Der übrige innere Randbereich 12 der Kapsulorhexis befindet sich, wie beim Ausführungsbeispiel der Fig. 1 und 2 auf der Oberfläche des optischen Linsenteils 1. Bezüglich der durch den optischen Linsenteil 1 gelegten Mittelebene 7 befinden sich sowohl die Stufen 4 bzw. Nuten 5 als auch die Oberfläche des optischen Linsenteils 1, auf welcher der Rand 12 der Kapsulorhexis aufliegt, auf der gleichen Seite (unterhalb der Mittellinie 7 in Fig. 14).

Aus optischen Linsenteil und plattenförmigen Haptikbügeln gebildete einstückige Linsenkörper können auch bei Intraokularlinsen zur Anwendung kommen, bei denen der optische Linsenteil in die Kapsulorhexis nicht eingeknöpft wird, d.h. bei solchen Intraokularlinsen, bei denen hierfür vorgesehene diametrale Nuten 5 oder die umlaufende Nut 6 (Fig. 9 und 10) oder die diametralen Stufen 4 nicht vorgesehen sind. Auch bei diesen Linsen ergibt sich der Vorteil, dass die Linsenoberfläche nicht poliert werden muss und ferner dass der Linsenkörper bestehend aus Haptik und optischem Linsenteil einstückig aus einem Linsenrohling hergestellt werden kann. Erreicht wird dies durch die teilzylindrisch ausgebildete Übergangsfläche 8 zwischen der plattenförmigen Haptik 2 und der Oberfläche des optischen Linsenteiles 1.

Bezugszeichenliste
- 1: optischer Linsenteil
- 2: Haptikbügel
- 3: Verbindungsstellen
- 4: Stufen
- 5: diametrale Nuten
- 6: umlaufende Nut
- 7: Mittelebene des Linsenkörpers
- 8: Übergangsfläche
- 9: Übergangsfläche
- 10: Ränder der Kapsulorhexis
- 11: sichelförmige Kapselrandbereiche
- 12: Ränder der Kapsulorhexis
- 13: Kapselwand
- 14: Mittellinie

## Patentansprüche

1. Intraokularlinse, welche in oder durch eine Kapsulorhexis im Auge zu implantieren ist, mit einem aus einem optischen Linsenteil (1) und einem haptischen Linsenteil bestehenden Linsenkörper, wobei der haptische Linsenteil zwei von Verbindungsstellen (3) an der Oberfläche des optischen Linsenteils (1) sich wegerstreckende Haptikbügel (2) aufweist und im Bereich der Verbindungsstellen (3) am Linsenkörper Eingriffsstellen (4; 5; 6), welche zum jeweiligen Aufnehmen eines Teils des die Kapsulorhexis umgebenden Randbereichs der Linsenkapsel geeignet sind, gebildet sind, **dadurch gekennzeichnet, dass** die zwischen den Haptikbügeln (2) liegenden und dem umlaufenden Linsenrand benachbarten Oberflächenteile des optischen Linsenteils (1) ausgebildet sind, die Kapsulorhexis umgebende Kapselrandbereiche, welche zwischen den in den Eingriffsstellen (4; 5; 6) liegenden Teilen der Kapsulorhexis liegen, mit mechanischer Spannung aufliegend aufzunehmen.

2. Intraokularlinse nach Anspruch 1, **dadurch gekennzeichnet, dass** am Linsenkörper zwei diametral zueinander liegende Eingriffsstellen (4; 5) vorgesehen sind.

3. Intraokularlinse nach Anspruch 1 oder 2, **dadurch gekennzeichnet, dass** die Eingriffsstellen (4; 5; 6) am optischen Linsenteil (1) vorgesehen sind.

4. Intraokularlinse nach einem der Ansprüche 1 bis 3, **dadurch gekennzeichnet, dass** die jeweilige Eingriffsstelle als Stufe (4) ausgebildet ist.

5. Intraokularlinse nach einem der Ansprüche 1 bis 3, **dadurch gekennzeichnet, dass** die jeweilige Eingriffsstelle als zwischen dem Haptikbügel (2) und dem Linsenkörper, insbesondere dem optischen Linsenteil (1) liegende Kerbe oder Nut (5) ausgebildet ist.

6. Intraokularlinse nach einem der Ansprüche 1 bis 5, **dadurch gekennzeichnet, dass** die Eingriffsstellen (4; 5) außerhalb einer durch den äußeren Umfangsrand des optischen Linsenteils (1) gelegten Mittelebene (7) liegen.

7. Intraokularlinse nach einem der Ansprüche 1 bis 6, **dadurch gekennzeichnet, dass** bezüglich der Mittelebene (7) die Eingriffsstellen (4; 5) und eine zum Anlegen des restlichen Teils des die Kapsulorhexis umgebenden Randbereichs (11) der Linsenkapselwand (13) ausgebildete Oberfläche des optischen Linsenteils (1) auf der gleichen Seite der Mittelebene (7) vorgesehen sind.

8. Intraokularlinse nach einem der Ansprüche 1 bis 5, **dadurch gekennzeichnet, dass** die Eingriffsstellen (6) in der Mittelebene liegen.

9. Intraokularlinse nach einem der Ansprüche 1 bis 5 und 8, **dadurch gekennzeichnet, dass** die Eingriffsstellen als umlaufende Eingriffsstelle, insbesondere Nut (6) am optischen Lisnenteil (1) ausgebildet sind.

10. Intraokularlinse nach einem der Ansprüche 1 bis 9, **dadurch gekennzeichnet, dass** der in eine Kapsulorhexis in der Vorderkapsel (vordere Linsenkapselwand) zu implantierende Linsenkörper Haptikbügel (2) aufweist, die zur Abstützung im Sulcus des Auges ausgebildet sind.

11. Intraokularlinse nach einem der Ansprüche 1 bis 9, **dadurch gekennzeichnet, dass** der in eine Kapsulorhexis in der Hinterkapsel (hintere Linsenkapselwand) einzusetzende Linsenkörper Haptikbügel (2) aufweist, die zur Abstützung im Kapselsack des Auges ausgebildet sind.

12. Intraokularlinse nach einem der Ansprüche 1 bis 11, **dadurch gekennzeichnet, dass** der aus Haptik und optischen Linsenteil bestehende Linsenkörper als gedrehter Linsenkörper ausgebildet ist.

13. Intraokularlinse nach einem der Ansprüche 1 bis 12, **dadurch gekennzeichnet, dass** die Haptikbügel (2) fadenförmig oder plattenförmig ausgebildet sind.

14. Intraokularlinse mit einem Haptikteil und einem optischen Linsenteil nach einem der Ansprüche 1 bis 13, **dadurch gekennzeichnet, dass** Übergangsflächen (8) zwischen dem optischen Linsenteil (1) und den plattenförmig ausgebildeten Haptikbügeln (2) teilzylindrische Oberflächen aufweisen.

15. Intraokularlinse nach Anspruch 14, **dadurch gekennzeichnet, dass** die Zylinderachsen der teilzylindrischen Oberflächen parallel zu einer durch die Mitte des Linsenkörpers verlaufenden Mittellinie (14) sind.

16. Intraokularlinse nach einem der Ansprüche 1 bis 15, **dadurch gekennzeichnet, dass** der optische Linsenteil (1) und die plattenförmigen Haptikbügel (2) als gedrehter Linsenkörper einstückig ausgebildet sind.

## Claims

1. Intraocular lens to be implanted into or through a capsulorhexis in an eye, having a lens body consisting of an optical lens portion (1) and a haptic lens portion, wherein the haptic lens portion comprises two haptic retainers (2) extending from connecting areas (3) on the surface of the optical lens portion (1), and at the lens body in the region of the connecting areas (3) engagement areas (4; 5; 6) are formed which are adapted to respectively receive a portion of the capsulorhexis-surrounding periphery of the lens capsule, **characterized in that**
the surface portions of the optical lens portion (1) lying between the haptic retainers (2) and being adjacent to the circumferential lens edge are adapted to receive, with mechanical tension, capsule edge areas surrounding the capsulorhexis which are arranged between the portions of the capsulorhexis lying in the engagement areas (4; 5; 6).

2. Intraocular lens according to claim 1, **characterized in that** two mutual diametric engagement areas (4; 5) are provided on the lens body.

3. Intraocular lens according to claim 1 or 2, **characterized in that** the engagement areas (4; 5; 6) are provided at the optical lens portion (1).

4. Intraocular lens according to any one of the claims 1 to 3, **characterized in that** the respective engagement area is formed as a step (4).

5. Intraocular lens according to any one of the claims 1 to 3, **characterized in that** the respective engagement area is formed as a notch or groove (5) arranged between the haptic retainers (2) and the lens body, in particular the optical lens portion (1).

6. Intraocular lens according to any one of the claims 1 to 5, **characterized in that** the engagement areas (4; 5) are arranged outside of a center plane (7) placed through the outer peripheral edge of the optical lens portion (1).

7. Intraocular lens according to any one of the claims 1 to 6, **characterized in that**, with respect to the center plane (7), the engagement areas (4; 5) and a surface of the optical lens portion (1) which is provided for joining the remaining portion of the capsulorhexis-surrounding edge area (11) of the lens capsule wall (13) are provided on the same side of the center plane (7).

8. Intraocular lens according to any one of the claims 1 to 5, **characterized in that** the engagement areas (6) are arranged in the center plane.

9. Intraocular lens according to any one of the claims 1 to 5 and 8, **characterized in that** the engagement areas are formed as circumferential engagement area, in particular as a groove at the optical lens portion (1).

10. Intraocular lens according to any one of the claims 1 to 9, **characterized in that** the lens body to be implanted into a capsulorhexis in the front capsule (front lens capsule wall) comprises haptic retainers (2) which are formed for a support in the sulcus of the eye.

11. Intraocular lens according to any one of the claims 1 to 9, **characterized in that** the lens body to be implanted into a capsulorhexis in the rear capsule (rear lens capsule wall) comprises haptic retainers (2) which are formed for a support in the capsular bag of the eye.

12. Intraocular lens according to any one of the claims 1 to 1, **characterized in that** the lens body consisting of haptics and an optical lens portion is formed as a turned lens body.

13. Intraocular lens according to any one of the claims 1 to 12, **characterized in that** the haptic retainers (2) are formed filament-like or plate-like.

14. Intraocular lens having a haptic portion and an optical lens portion according to any one of the claims 1 to 13, **characterized in that** junction faces (8) between the optical lens portion (1) and the plate-like formed haptic retainers (2) have partial cylindrical surfaces.

15. Intraocular lens according to claim 14, **characterized in that** the cylinder axes of the partial cylindrical surfaces are parallel to a center line (14) extending through the center of the lens body.

16. Intraocular lens according to any one of the claims 1 to 15, **characterized in that** the optical lens portion (1) and the plate-like haptic retainers (2) are integrally formed as a turned lens body.

## Revendications

1. Lentille intraoculaire qui est destinée à être implantée dans l'oeil dans ou à travers un capsulorhexis, comportant un corps de lentille formé par une partie de lentille optique (1) et une partie de lentille haptique, ladite partie de lentille haptique comportant deux branches haptiques (2), qui s'étendent en s'éloignant des lieux de liaison (3) à la surface de la partie de lentille optique (1), et, dans la zone des lieux de liaison (3) au corps de lentille, des zones de prise (4 ; 5 ; 6) qui sont propres à recevoir chacune une partie de la zone périphérique de la capsule cristalline entourant le capsulorhexis, **caractérisée en ce que** les parties de surface de la partie de lentille optique (1), qui sont situées entre les branches haptiques (2) et qui sont adjacentes au bord périphérique de la lentille, sont réalisées pour recevoir en appui avec une contrainte mécanique les zones périphériques de la capsule entourant le capsulorhexis, lesquelles sont situées entre les parties du capsulorhexis qui sont situées dans les zones de prise (4 ; 5 ; 6).

2. Lentille intraoculaire selon la revendication 1, **caractérisée en ce que** deux zones de prise (4 ; 5) au corps de lentille diamétralement opposées l'une à l'autre sont prévues.

3. Lentille intraoculaire selon la revendication 1 ou 2, **caractérisée en ce que** les zones de prise (4 ; 5 ; 6) sont prévues à la partie de lentille optique (1).

4. Lentille intraoculaire selon l'une quelconque des revendications 1 à 3, **caractérisée en ce que** les zones de prise respectives sont réalisées sous la forme d'un gradin (4).

5. Lentille intraoculaire selon l'une quelconque des revendications 1 à 3, **caractérisée en ce que** les zones de prise respectives sont réalisées sous la forme d'une encoche ou d'une rainure (5) située entre la branche haptique (2) et le corps de lentille, en particulier la partie de lentille optique (1).

6. Lentille intraoculaire selon l'une quelconque des revendications 1 à 5, **caractérisée en ce que** les zones de prise (4 ; 5) sont situées en dehors d'un plan médian (7) passant à travers le bord périphérique extérieur de la partie de lentille optique (1).

7. Lentille intraoculaire selon l'une quelconque des revendications 1 à 6, **caractérisée en ce que** par rapport au plan médian (7), les zones de prise (4 ; 5) et une surface de la partie de lentille optique (1 réalisée pour recevoir en appui la partie restante de la zone périphérique (11) de la paroi (13) de la capsule cristalline entourant le capsulorhexis, sont prévues sur le même côté du plan médian (7).

8. Lentille intraoculaire selon l'une quelconque des revendications 1 à 5, **caractérisée en ce que** les zones de prise (6) sont situées dans le plan médian.

9. Lentille intraoculaire selon l'une quelconque des revendications 1 à 5 et 8, **caractérisée en ce que** les zones de prise sont réalisées sous forme de zones de prise périphériques, en particulier sous la forme d'une rainure (6) au niveau de la partie de lentille optique (1).

10. Lentille intraoculaire selon l'une quelconque des revendications 1 à 9, **caractérisée en ce que** le corps de lentille, destiné à être implanté dans un capsulorhexis dans la capsule antérieure (paroi antérieure de la capsule cristalline), comporte des branches haptiques (2) qui sont réalisées pour venir en appui dans le sillon de l'oeil.

11. Lentille intraoculaire selon l'une quelconque des revendications 1 à 9, **caractérisée en ce que** le corps de lentille, à implanter dans un capsulorhexis dans la capsule postérieure (paroi postérieure de la capsule cristalline), comporte des branches haptiques (2) qui sont réalisées pour venir en appui dans le sac capsulaire de l'oeil.

12. Lentille intraoculaire selon l'une quelconque des revendications 1 à 11, **caractérisée en ce que** le corps de lentille, formé par la partie de lentille haptique et la partie de lentille optique, est réalisé sous la forme d'un corps de lentille usiné au tour.

13. Lentille intraoculaire selon l'une quelconque des revendications 1 à 12, **caractérisée en ce que** les branches haptiques (2) sont réalisées en forme de fil ou en forme de plaque.

14. Lentille intraoculaire comportant une partie de lentille haptique et une partie de lentille optique selon l'une quelconque des revendications 1 à 13, **caractérisée en ce que** les surfaces de transition (8) entre la partie de lentille optique (1) et les branches haptiques (2) en forme de plaque comportent des surfaces partiellement cylindriques.

15. Lentille intraoculaire selon la revendication 14, **caractérisée en ce que** les axes des surfaces partiellement cylindriques sont parallèles à une ligne médiane (14) passant par le centre du corps de lentille.

16. Lentille intraoculaire selon l'une quelconque des revendications 1 à 15, **caractérisée en ce que** la partie de lentille optique (1) et les branches haptiques (2) en forme de plaque sont réalisées d'un seul tenant sous la forme d'un corps de lentille usiné au tour.
